# EUROPÄISCHE PATENTSCHRIFT

(11) **EP 3 021 756 B1**
(45) Veröffentlichungstag und Bekanntmachung des Hinweises auf die Patenterteilung: **29.05.2019**
(21) Anmeldenummer: 14750156.3
(22) Anmeldetag: 16.07.2014
(51) Int. Cl.: A61B 6/14

(54) **VERFAHREN ZUR DURCHFÜHRUNG EINER DREIDIMENSIONALEN RÖNTGENAUFNAHME**
METHOD FOR CAPTURING A THREE-DIMENSIONAL X-RAY IMAGE
PROCÉDÉ PERMETTANT D'EFFECTUER UNE RADIOGRAPHIE TRIDIMENSIONNELLE

(30) Priorität: 16.07.2013 DE 102013213876
(43) Veröffentlichungstag der Anmeldung: 25.05.2016
(73) Patentinhaber: Sirona Dental Systems GmbH, 64625 Bensheim (DE)
(72) Erfinder: LINDENBERG, Kai, 64395 Wersau (DE); WUNDRAK, Stefan, 64625 Bensheim (DE)
(74) Vertreter: Sommer, Peter
(86) Internationale Anmeldenummer: PCT/EP2014/065217
(87) Internationale Veröffentlichungsnummer: WO 2015/007765

(56) Entgegenhaltungen:
- DE-A1- 10 221 634
- DE-T5-112006 002 694

## Beschreibung

### Technisches Gebiet

Die Erfindung betrifft ein Verfahren zur Durchführung einer Röntgenaufnahme eines Objekts mittels eines Röntgensystems, umfassend eine Röntgenquelle, einen Röntgendetektor und eine Blendenmatrix, wobei die Blendenmatrix mehrere Blendenelemente aufweist, die in ihren Röntgenabsorptionseigenschaften steuerbar sind.

### Stand der Technik

Aus dem Stand der Technik sind mehrere Röntgensysteme bekannt, die eine Blendenmatrix mit mehreren Blendenelementen verwenden.

DE 699 10 524 T2 offenbart eine Röntgenstrahlung-Prüfungsvorrichtung umfassend einen Röntgenfilter aus mehreren Filterelementen, deren Absorptionsvermögen gegenüber Röntgenstrahlen durch Anpassung der Menge röntgenabsorbierender Flüssigkeit innerhalb einzelner Filterelemente justiert werden kann. Die Adhäsion der röntgenabsorbierenden Flüssigkeit an der Innenwand von Kapillarröhrchen solcher Elemente ist davon abhängig, welche elektrische Spannung an dem Kapillarröhrchen angelegt ist.

Aus dem Stand der Technik sind Röntgensysteme mit festen Blendenanordnungen bekannt, um einen aufzunehmenden Bereich festzulegen. Solche Blendensysteme können meist nur rechteckige Aufnahmebereiche einstellen.

Ein Nachteil solcher Systeme besteht also darin, dass ein rechteckiger Aufnahmebereich um das eigentliche Zielobjekt festgelegt wird und unnötigerweise Gewebe des Patienten um das Zielobjekt bestrahlt wird, so dass damit die Dosisbelastung erhöht wird.

Die DE 102 10 21 634 A1 beschreibt eine adaptive Bildinhaltsgesteuerte örtliche Strahlenschwächung zur Dosisreduzierung, wobei zur örtlichen Abschwächung matrixartig angeordnete flache Schwächungselemente, wie Metallstäbe oder Platten, mittels piezoelektrischer Aktoren im Strahlengang ausgerichtet werden.

Aus der DE 11 2006 002 694 T5 ist das computergestützte Auswählen eines aufzunehmenden Bereichs anhand einer Übersichtsaufnahme bekannt.

Die Aufgabe der vorliegenden Erfindung besteht also darin, ein Verfahren zur Durchführung einer Röntgenaufnahme bereitzustellen, welches eine dreidimensionale Röntgenaufnahme mit einer möglichst geringen Dosisbelastung ermöglicht.

### Darstellung der Erfindung

Die Erfindung betrifft ein Verfahren zur Durchführung einer dreidimensionalen Röntgenaufnahme eines Objekts mittels eines Röntgensystems, umfassend eine Röntgenquelle, einen Röntgendetektor und eine Blendenmatrix. Die Blendenmatrix weist dabei mehrere Blendenelemente auf, die in ihren Röntgenabsorptionseigenschaften steuerbar sind. In einem Verfahrensschritt wird mindestens ein erster aufzunehmender Bereich des Objekts festgelegt. In einem weiteren Verfahrensschritt werden unter Berücksichtigung des festgelegten ersten aufzunehmenden Bereichs Einstellungen für die einzelnen Blendenelemente der Blendenmatrix für verschiedene Umlaufpositionen geplant. Anschließend werden mehrere zweidimensionale Röntgenaufnahmen aus den geplanten Umlaufpositionen während mindestens eines partiellen Umlaufs mit den geplanten Einstellungen der Blendenelemente aufgenommen.

Aus den einzelnen zweidimensionalen Röntgenaufnahmen wird dann die gesamte dreidimensionale Röntgenaufnahme des ersten aufzunehmenden Bereichs erzeugt. Zusätzlich zum ersten aufzunehmenden Bereich wird mindestens ein zweiter aufzunehmender Bereich festgelegt, der vom ersten aufzunehmenden Bereich räumlich abgetrennt ist, wobei die Blendenelemente der Blendematrix so angesteuert werden, dass der erste aufzunehmende Bereich und mindestens der zweite aufzunehmende Bereich gleichzeitig aufgenommen werden, so dass zwei oder mehrere räumlich voneinander getrennte Bereiche gleichzeitig aufgenommen werden.

Die dreidimensionale Röntgenaufnahme kann beispielsweise eine DVT-Röntgenaufnahme oder eine CT-Röntgenaufnahme sein. Bei dem sogenannten digitalen Volumentomographieverfahren (DVT) oder bei dem sogenannten Computertomographie-Verfahren (CT) werden die Röntgenquelle und der Röntgendetektor in einer definierten Ebene um das aufzunehmende Objekt, wie ein Patientenkopf, bewegt, wobei aus verschiedenen Richtungen zweidimensionale Röntgenaufnahmen erzeugt werden, wobei im nächsten Schritt aus diesen Projektionsaufnahmen ein dreidimensionales Volumen berechnet wird.

Diese berechnete dreidimensionale Röntgenaufnahme kann dann in einer Benutzersoftware mittels einer Anzeigevorrichtung, wie eines Bildschirms, dargestellt werden. Die Bildgebung beruht also auf einer kontinuierlichen Aufnahme der Projektion aus unterschiedlichen Richtungen, wobei unter Verwendung eines Rekonstruktionsverfahrens die dreidimensionale Röntgenaufnahme berechnet wird, wobei den sogenannten Voxeln der dreidimensionalen Röntgenaufnahme die jeweiligen Röntgenabsorbtionswerte zugeordnet werden.

Die Blendenmatrix kann beispielsweise aus Blendenelementen bestehen, die nach dem elektrokapillarischen Prinzip funktionieren, wobei die Blendenmatrix ein Bündel mit einer sehr großen Anzahl von Kapillarröhrchen, die alle jeweils an einem Ende eine Verbindung zu einer röntgenabsorbierenden Flüssigkeit aufweisen, wobei die Adhäsion der röntgenabsorbierenden Flüssigkeit an der Innenwand eines solchen Kapillarröhrchens davon abhängig ist, welche elektrische Spannung an dem entsprechenden Kapillarröhrchen angelegt ist. Damit können die einzelnen Kapillarröhrchen bezüglich ihres Röntgenabsorbtionsvermögens gesteuert werden. Die Blendenmatrix kann beispielsweise eine 100 x 100 Matrixanordnung mit den Abmessungen 5 cm x 5 cm umfassen. Eine noch höhere Auflösung beispielsweise bei einer 200 x 200 Matrixanordnung ermöglicht eine höhere Auflösung der Blendenmatrix.

Der aufzunehmende Bereich kann beispielsweise der gesamte Oberkiefer, der gesamte Unterkiefer, das linke Kiefergelenk und/oder das rechte Kiefergelenk sein. Der festgelegte aufzunehmende Bereich kann jedoch auch nur eine Gruppe von einzelnen Zähnen umfassen.

Die Einstellungen der Blendenmatrix für die einzelnen Umlaufpositionen werden also computergestützt berechnet, so dass beispielsweise Röntgenstrahlen der Röntgenquelle, die auf die umliegenden Bereiche außerhalb des festgelegten aufzunehmenden Bereichs projiziert werden, mittels der entsprechenden Blendenelemente vollständig ausgeblendet werden, wobei lediglich die Röntgenstrahlen, die auf den aufzunehmenden Bereich projiziert werden, durchgelassen werden. Die Einstellungen der Blendenelemente umfassen also die einzustellenden Röntgenabsorptionswerte für die einzelnen Blendenelemente für jede der Umlaufpositionen.

Bei einem partiellen Umlauf von beispielsweise 180° können die zweidimensionalen Röntgenaufnahmen aus den Umlaufpositionen in Schritten von 1°, also für 180 verschiedene Umlaufpositionen, erfolgen. Ein kleinerer Umlauf von beispielsweise 90° oder auch ein größerer Umlauf mit 270° in 0,5°-Schritten ist ebenfalls möglich, wobei eine höhere Anzahl von zweidimensionalen Röntgenaufnahmen zu weniger Artefakten und damit zu einer besseren Aufnahmequalität der rekonstruierten dreidimensionalen Röntgenaufnahme führt. Dies ist jedoch bei gleichbleibender Einzelbilddosis auch mit einer höheren Dosisbelastung verbunden ist.

Ein Vorteil des vorliegenden Verfahrens besteht darin, dass im Vergleich zu herkömmlichen Verfahren lediglich der aufzunehmende Bereich mit Röntgenstrahlung bestrahlt wird und damit die Dosisbelastung minimiert wird. Das den aufzunehmenden Bereich umgebende Gewebe wird dabei also nicht bestrahlt.

Ein weiterer Vorteil des vorliegenden Verfahrens besteht darin, dass mehrere nicht zusammenhängende Bereiche mit minimaler Dosis erfasst werden können. Im Gegensatz dazu müssten bei herkömmlichen Verfahren voneinander abgetrennte Bereiche nacheinander in mehreren Aufnahmen vermessen werden.

Ein weiterer Vorteil besteht darin, dass bei der Aufnahme eines so genannten Sub-Volumens eine Gantry-Mechanik einer herkömmlichen Röntgenvorrichtung deutlich vereinfacht hergestellt werden kann, da die Verschiebung des Volumenzentrums über die Blendenmatrix erfolgt und nicht über Aktuatoren, die am Gantry angeordnet sind.

Ein weiterer Vorteil besteht darin, dass zwei oder mehrere räumlich voneinander getrennte aufzunehmende Bereiche bzw. Volumina gleichzeitig in einer dreidimensionalen Röntgenaufnahme aufgenommen werden. Die Blendenmatrix wird also so angesteuert, dass nur Strahlenbündel zum Objekt gelangen, die die festgelegten Bereiche beziehungsweise Volumina passieren. Die übrigen Strahlenbündel werden ausgeblendet, so dass die gesamte Dosis der Röntgenaufnahme im Vergleich zu herkömmlichen Verfahren, bei denen jeder Bereich einzeln vermessen werden muss, vermindert wird.

Vorteilhafterweise kann der aufzunehmende Bereich in einer vor der Aufnahme durchgeführten Übersichtsaufnahme festgelegt werden.

Die Übersichtsaufnahme des Objekts liegt bereits vor und wurde vor der Durchführung des erfinderischen Verfahrens aufgenommen.

Der aufzunehmende Bereich kann also beispielsweise computergestützt mittels einer Anzeigevorrichtung, wie eines Monitors, manuell oder automatisch in diese Übersichtsaufnahme festgelegt werden.

Vorteilhafterweise kann die Übersichtsaufnahme eine zweidimensionale optische Aufnahme, eine dreidimensionale optische Aufnahme, eine dreidimensionale Röntgenaufnahme oder eine zweidimensionale Röntgenaufnahme sein.

Die Übersichtsaufnahme kann also beispielsweise eine dreidimensionale optische Aufnahme, wie eine DVT-Aufnahme oder eine CT-Aufnahme, eine herkömmliche zweidimensionale Röntgenaufnahme oder auch eine dreidimensionale optische Aufnahme sein, wobei die dreidimensionale optische Aufnahme beispielsweise mittels einer dentalen Kamera aufgenommen werden kann, die auf einem Streifenprojektionsverfahren beruht.

Die Übersichtsaufnahme kann beispielsweise auch eine optische Einzelaufnahme oder eine Videoaufnahme sein, die das aufzunehmende Objekt umfasst und mittels einer herkömmlichen Videokamera oder auch einer Stereo-Videokamera aufgenommen wurde.

Vorteilhafterweise kann der aufzunehmende Bereich manuell mittels eines Computers unter Verwendung von Eingabemitteln durch einen Benutzer in der Übersichtsaufnahme festgelegt werden.

Dadurch kann der Benutzer computergestützt mittels der Anzeigevorrichtung, wie eines Monitors, unter Verwendung von Eingabemitteln, wie einer Tastatur oder einer Maus, den betreffenden aufzunehmenden Bereich in der Übersichtsaufnahme auswählen.

Vorteilhafterweise kann der aufzunehmende Bereich automatisch mittels eines Computers in der Übersichtsaufnahme festgelegt werden, wobei bestimmte vordefinierte anatomische Bereiche mittels eines computergestützten Suchalgorithmus erkannt werden.

Der verwendete computergestützte Suchalgorithmus kann ein herkömmlicher Mustererkennungsalgorithmus und/oder ein Segmentierungsalgorithmus sein.

Bei bekannten Suchalgorithmen werden Objekte segmentiert und nach übereinstimmenden Strukturen durchsucht. Ein Mustererkennungsverfahren kann beispielsweise folgende Schritte umfassen, nämlich die Vorverarbeitung, die Merkmalsgewinnung, die Merkmalsreduktion und die Klassifizierung der Merkmale. Bei der Verarbeitung werden unerwünschte oder irrelevante Bestandteile der Bilddaten entfernt. Bei der Merkmalsgewinnung werden bestimmte Merkmale aus den Bilddaten durch Vergleich mit bekannten Mustern einer Datenbank, wie einer Datenbank von charakteristischen Zähnen oder Kieferknochen, gewonnen. Der automatische Vergleich erfolgt unter Anwendung von Transformationsfunktionen und Skalierungen, wobei durch Berechnung einer Varianz zwischen einem Muster aus den Bilddaten und einem erweiterten Muster aus der Datenbank ein Vergleichsfaktor berechnet wird. Bei der Merkmalsreduktion wird geprüft, welche Merkmale für die Klassentrennung relevant sind und welche weggelassen werden können. Insbesondere die gewonnen Muster von Zähnen und des Kieferknochens sind für dieses Verfahren relevant, wobei die gewonnen Merkmale unbeachtet bleiben können. Im letzten Schritt der Klassifizierung werden die wesentlichen erkannten Merkmale, wie Zähne und charakteristische Formgebungen des Kieferknochens, in zusammengehörige Klassen, wie Schneidezähne, Backenzähne, Zahnwurzeln und Kieferknochen unterteilt. Bei der Merkmalsgewinnung können bekannte Verfahren, wie die Rasteranalyse, die Cluster-Analyse und das Pattern-Matching, zur Anwendung kommen.

Vorteilhafterweise kann der aufzunehmende Bereich bei einer Vorauswahl festgelegt werden, wobei in einem zusätzlichen Verfahrensschritt anhand von bekannten Lagebeziehungen der Röntgenquelle, des Röntgendetektors und der Blendenmatrix relativ zueinander und anhand von Abmessungen eines in einem Datenspeicher abgelegten Modells eines Patienten eine bestimmte anatomische Struktur des Patientenkopfes als der aufzunehmende Bereich festgelegt wird.

Dadurch ist keine vorangehende Übersichtsaufnahme notwendig, denn zur Festlegung des aufzunehmenden Bereichs wird ein in der Datenbank abgelegter Musterkopf verwendet. Dabei kann beispielsweise ein Oberkiefer oder ein Unterkiefer des Musterkopfes ausgewählt werden, der dem Verlauf des aufzunehmenden Oberkiefers bzw. Unterkiefers möglichst genau entspricht.

Vorteilhafterweise kann der aufzunehmende Bereich mindestens eine anatomische Struktur umfassen.

Dadurch umfasst der aufzunehmende Bereich mindestens eine anatomische Struktur, wie einen Oberkiefer, einen Unterkiefer oder auch eine Gruppe von Zähnen.

Vorteilhafterweise kann die anatomische Struktur ein Oberkiefer, ein Unterkiefer, ein rechtes Kiefergelenk und/oder ein linkes Kiefergelenk beziehungsweise eine Teilstruktur dessen sein.

Dadurch wird also lediglich die jeweils aufzunehmende anatomische Struktur ausgewählt.

Vorteilhafterweise können die Einstellungen für die Blendenelemente der Blendenmatrix während des Umlaufs an eine aufzunehmende anatomische Struktur angepasst werden, wobei die aufzunehmende anatomische Struktur, beispielsweise der Unterkiefer, in mindestens einer vorangehenden zweidimensionalen Röntgenaufnahme einer vorangehenden Umlaufposition erkannt wird und anschließend die Einstellungen der Blendenelemente für eine nachfolgende Röntgenaufnahme aus einer nachfolgenden Umlaufposition an die Abmessungen der erkannten anatomischen Struktur aus der vorangehenden Röntgenaufnahme der vorangehenden Umlaufposition angepasst wird.

Die Einstellungen der Blendenelemente werden also für jede Umlaufposition abhängig von der zweidimensionalen Röntgenaufnahme der vorherigen Umlaufposition berechnet. Die Berechnung der Einstellungen für die einzelnen Blendenelemente für die neue Umlaufposition kann unter Verwendung eines Rekonstruktionsverfahrens erfolgen. Anhand der bekannten Lagebeziehungen zwischen der Röntgenquelle, dem Röntgendetektor, der Blendenmatrix und dem festgelegten aufzunehmenden Bereich wird also berechnet, welche Blendenelemente in der neuen Umlaufposition die Röntgenstrahlen ausblenden bzw. durchlassen sollen.

Bei der Berechnung der Einstellungen der Blendenelemente für die neue Umlaufposition kann auch ein Segmentierungsverfahren zum Erkennen der festgelegten anatomischen Struktur in der Röntgenaufnahme der vorangehenden Umlaufposition angewendet werden. Dadurch wird also noch während des Umlaufs bei jeder Umlaufposition die ausgewählte anatomische Struktur gesucht und die Blendenelemente entsprechend eingestellt. Dies hat den Vorteil, dass, falls die anatomische Struktur in einer zweidimensionalen Röntgenaufnahme als Übersichtsaufnahme ausgewählt wurde und daher die Abmessungen der ausgewählten anatomischen Struktur lediglich aus einer Richtung bekannt sind, die ausgewählte anatomische Struktur Schritt für Schritt während des Umlaufs für jede Umlaufposition in den aufgenommenen zweidimensionalen Röntgenaufnahmen bestimmt wird und die Blendenmatrix entsprechend angepasst wird. Dabei können auch geringfügige Bewegungen des Objekts, wie eines Patientenkopfes, während des Umlaufs relativ zum Röntgensystem ausgeglichen werden. Der Vorteil besteht also darin, dass die Abmessungen der aufzunehmenden anatomischen Struktur, wie des Oberkiefers oder des Unterkiefers, nicht vollständig bekannt sein müssen und erst während der Aufnahme Schrittweise für jede Umlaufposition bestimmt werden.

Vorteilhafterweise kann die Anpassung der Einstellungen für die Blendenelemente der Blendenmatrix an die anatomische Struktur schrittweise, Aufnahme für Aufnahme, für jede Umlaufposition erfolgen.

Dadurch erfolgt die Anpassung der Einstellungen der Blendenmatrix schrittweise für jede Umlaufposition.

Vorteilhafterweise kann die Blendenmatrix eine Anordnung von in einer Ebene angeordneten Blendenelementen sein.

Die Blendenmatrix kann also beispielsweise eine Blendenanordnung von 100 x 100 Blendenelementen aufweisen.

Vorteilhafterweise kann die Blendenmatrix eine zeilenförmige Anordnung von in einer Reihe angeordneten Blendenelementen sein.

Die Blendenmatrix kann beispielsweise eine zeilenförmige Anordnung von 100 Blendenelementen aufweisen.

### Kurzbeschreibung der Zeichnungen

Die Erfindung wird anhand der Zeichnungen erläutert. Es zeigt, die
- Fig. 1: eine Skizze zur Verdeutlichung des Verfahrens zur Durchführung einer dreidimensionalen Röntgenaufnahme, die
- Fig. 2: eine Skizze der Blendenmatrix mit den Blendenelementen in einer ersten Umlaufposition, die
- Fig. 3: eine Skizze der Blendenmatrix mit den Blendenelementen in einer zweiten Umlaufposition, die
- Fig. 4: eine Skizze der Blendenmatrix mit den Blendenelementen in einer dritten Umlaufposition, die
- Fig. 5: eine Skizze der Blendenmatrix mit den Blendenelementen in einer vierten Umlaufposition.

### Ausführungsbeispiel

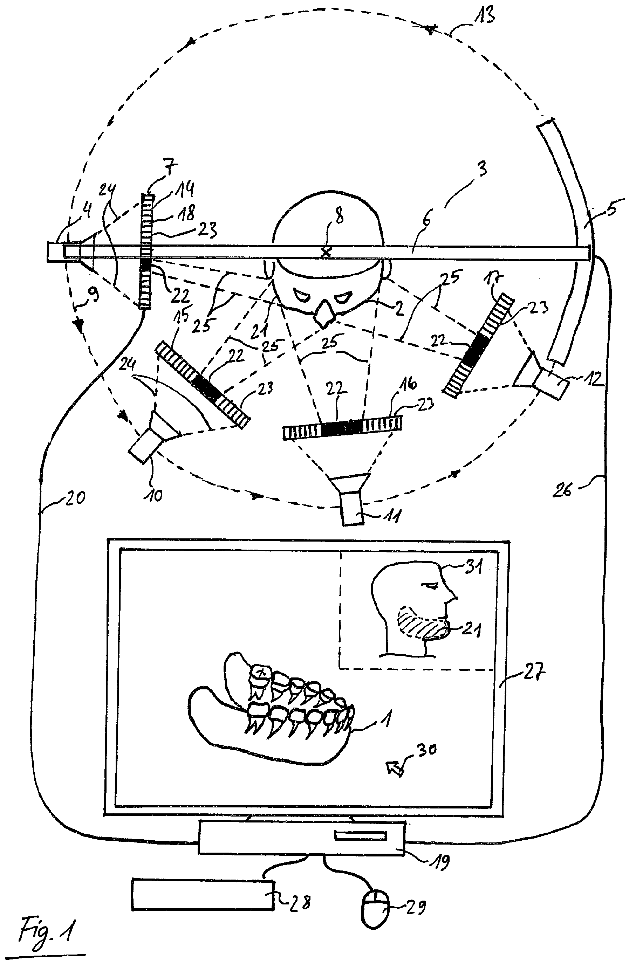

Die Fig. 1 zeigt eine Skizze zur Verdeutlichung des Verfahrens zur Durchführung einer dreidimensionalen Röntgenaufnahme 1 eines Objekts 2, wie eines Patientenkopfes, mittels eines Röntgensystems 3,umfassend eine Röntgenquelle 4, einen rechteckigen Röntgendetektor 5, einen Tragarm 6 und eine Blendenmatrix 7. Die Röntgenquelle 4 und der Röntgendetektor 5 sind an den gegenüberliegenden Enden des Tragarms 6 angeordnet, der im Röntgensystem 3 so gelagert ist, dass der Tragarm 6 zusammen mit der Röntgenquelle 4, dem Röntgendetektor 5 und der am Tragarm 6 befestigten Blendenmatrix 7 um eine gemeinsame Drehachse 8, die als ein Kreuz dargestellt ist, rotiert wird. Während eines zumindest teilweisen Umlaufs wird die Röntgenquelle 4 entlang einer ersten kreisförmigen Trajektorie 9 zu einer zweiten Umlaufposition 10, zu einer dritten Umlaufposition 11 und anschließend zu einer vierten Umlaufposition 12 bewegt. Der Röntgendetektor 5 wird entsprechend entlang einer zweiten Trajektorie 13 rotiert. Die Blendenmatrix 7 wird also zusammen mit dem Tragarm 6 ebenfalls ausgehend von einer ersten Umlaufposition 14 zu einer zweiten Umlaufposition 15, zu einer dritten Umlaufposition 16 und anschließend zu einer vierten Umlaufposition 17 bewegt.

Die Blendenmatrix 7 weist mehrere Blendenelemente 18 auf, die in ihren Röntgenabsorbtionseigenschaften steuerbar sind. Die Steuerung erfolgt mittels eines Computers 19, der die Steuerdaten an die Blendenmatrix 7 mittels einer Kabelverbindung 20 übermittelt. Der Datentransfer zwischen der Datenmatrix 7 und dem Computer 19 kann auch drahtlos erfolgen. Die Blendenelemente 18 der Blendenmatrix 7 können beispielsweise mit einer röntgenabsorbierenden Flüssigkeit gefüllte Kapillarröhrchen sein, wobei die Adhäsion der röntgenabsorbierenden Flüssigkeit an der Innenwand der Kapillarröhrchen davon abhängig ist, welche elektrische Spannung an den entsprechenden Kapillarröhrchen angelegt ist. Im ersten Schritt des Verfahrens wird ein aufzunehmender Bereich 21, der gestrichelt dargestellt ist und im vorliegenden Fall einem Unterkiefer entspricht, in einer Übersichtsaufnahme 31 oder alternativ an einem Musterkopf aus einer Datenbank ausgewählt. Die Übersichtsaufnahme 22 kann beispielsweise eine zweidimensionale Röntgenaufnahme, eine dreidimensionale optische Aufnahme oder auch eine dreidimensionale Röntgenaufnahme des Patienten sein, die vor der Durchführung des Verfahrens aufgenommen wurde. Anschließend werden für die erste Umlaufposition 14 unter Berücksichtigung des festgelegten aufzunehmenden Bereichs 21, nämlich des Unterkiefers, Einstellungen für die einzelnen Blendenelemente 18 der Blendenmatrix 7 mittels des Computers 19 berechnet. Dabei werden die Blendenelemente 18 eines ersten Blendenmatrixbereichs 22, der schwarz markiert ist, so angesteuert, dass sie einen möglichst geringen Röntgenabsorbtionswert haben, wobei die übrigen Blendenelemente 18 innerhalb eines zweiten Blendenmatrixbereichs 23 außerhalb des ersten Blendenmatrixbereichs 22 so angesteuert werden, dass der Röntgenabsorbtionswert möglichst hoch ist. Damit werden die von der Röntgenquelle 4 ausgestrahlten Röntgenstrahlen 24 innerhalb des zweiten Blendenmatrixbereichs 23 ausgeblendet und innerhalb des ersten Blendenmatrixbereichs 22 zum Objekt 2 durchgelassen. Dadurch wird also lediglich der festgelegte aufzunehmende Bereich 21, nämlich im dargestellten Fall der Unterkiefer, mit den durchgelassenen Aufnahmeröntgenstrahlen 25 bestrahlt. Die mittels des Röntgendetektors 5 aufgenommenen Röntgendaten einer ersten zweidimensionalen Röntgenaufnahme werden dann mittels einer Kabelverbindung 26 oder alternativ auch drahtlos an den Computer 19 übermittelt. Die erste zweidimensionale Röntgenaufnahme, die aus der ersten Umlaufposition 14 aufgenommen wurde, kann dann unter Anwendung von computergestützten Suchalgorithmen nach dem festgelegten aufzunehmenden Bereich 21, nämlich nach dem Unterkiefer, durchsucht werden. Die Abmessungen bzw. Umrisse des Unterkiefers 21 aus der ersten zweidimensionalen Röntgenaufnahme werden dann zur Berechnung der Einstellungen der Röntgenmatrix 7 für die zweite Umlaufposition 15 verwendet. Anschließend wird die Röntgenquelle 4 zusammen mit der Röntgenmatrix 7 in die zweite Umlaufposition 15 bewegt. Die Blendenelemente 18 der Blendenmatrix 7 werden dann so angesteuert, dass der erste Blendenmatrixbereich 22 röntgenstrahlendurchlässig ist und der zweite Blendenmatrixbereich 23 die Röntgenstrahlen ausblendet, so dass lediglich die Aufnahmeröntgenstrahlen 25 zum Objekt 2 durchgelassen werden. Der Röntgendetektor 5 nimmt dann in der zweiten Umlaufposition 15 eine zweite zweidimensionale Röntgenaufnahme auf und übermittelt die Röntgendaten an den Computer 19. Darin wird wieder der Unterkiefer 21 unter Verwendung der Suchalgorithmen erkannt. Anschließend werden die Einstellungen der Blendenmatrix 7 für die dritte Umlaufposition 16 berechnet. Auf dieselbe Weise erfolgen auch die dritte zweidimensionale Röntgenaufnahme aus der dritten Umlaufposition 16 und die vierte zweidimensionale Röntgenaufnahme aus der vierten Umlaufposition 17. Im letzten Verfahrensschritt wird aus den einzelnen zweidimensionalen Röntgenaufnahmen der Umlaufpositionen 14, 15, 16 und 17 durch Rekonstruktion die gesamte dreidimensionale Röntgenaufnahme 1 des Unterkiefers 21 erzeugt, die mittels eines Monitors 27 dargestellt wird. Die Auswahl des aufzunehmenden Bereichs 21 in der Übersichtsaufnahme 22 sowie die Navigation in der dreidimensionalen Röntgenaufnahme 1 kann manuell durch den Benutzer unter Verwendung von Eingabemitteln, wie einer Tastatur 28 und einer Maus 29, durch einen Cursor 30 erfolgen. Nach diesem Verfahren wird also die dreidimensionale Röntgenaufnahme 1 des aufzunehmenden Bereichs 21, nämlich des Unterkiefers, erzeugt, wobei das umgebende Gewebe des Unterkiefers nicht bestrahlt wird und damit die Dosisbelastung des Patienten minimiert wird.

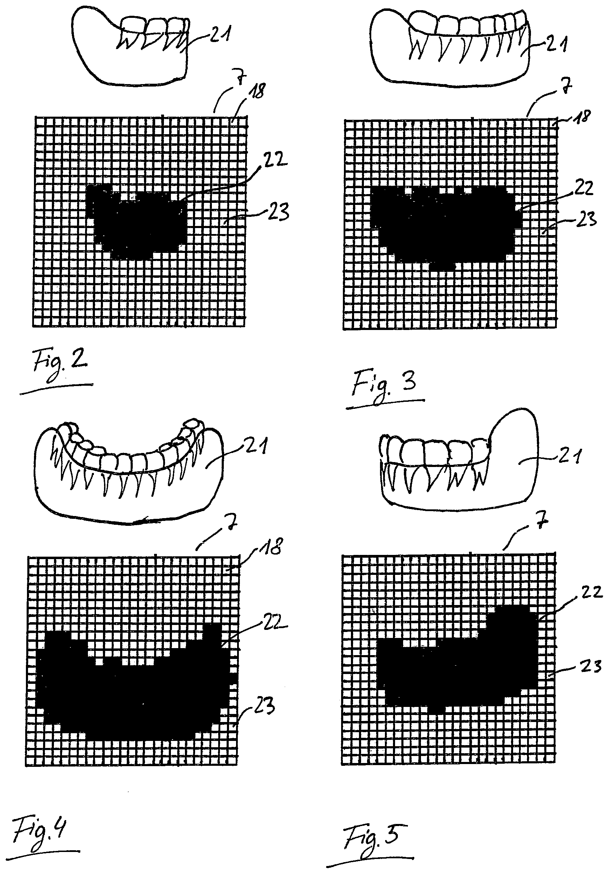

Die Fig. 2 zeigt eine Skizze der Blendenmatrix 7 mit den Blendenelementen 18, wobei die Blendenanordnung eine rechteckige Anordnung von 25 x 25 Blendenelementen darstellt. Eine Blendenanordnung von 100 x 100 oder noch mehr Blendenelementen kann ebenfalls verwendet werden und hat den Vorteil einer höheren Auflösung der Blendenmatrix 7.

Der aufzunehmende Bereich 21, nämlich der Unterkiefer, ist aus der Blickrichtung der ersten Umlaufposition 14 aus Fig. 1 dargestellt. Die Blendenmatrix 7 wurde so angesteuert, dass die Blendenelemente 18 innerhalb des schwarz markierten ersten Blendenmatrixbereichs 22 röntgendurchlässig sind und die übrigen Blendenelementen 18 innerhalb des zweiten Blendenmatrixbereichs 23 die Röntgenstrahlen ausblenden.

Die Fig. 3 zeigt den Unterkiefer 21 und die Blendenmatrix 7 in der zweiten Umlaufposition 15, die Fig. 4 in der dritten Umlaufposition 16 und Fig. 5 in der vierten Umlaufposition 17. Damit wird skizzenhaft verdeutlicht, auf welche Art und Weise die Blendenmatrix während des Umlaufs um das Objekt 2 angesteuert wird. Die zweidimensionalen Röntgenaufnahmen und die Verstellungen der Blendenmatrix 7 erfolgen also für eine Vielzahl von Umlaufpositionen 14, 15, 16 und 17, wobei die Umlaufposition in Schritten von beispielsweise 1° bis 5° verstellt werden kann. Für eine dreidimensionale Röntgenaufaufnahme ist ein partieller Umlauf beispielsweise zwischen 60° und 180° ausreichend.

### Bezugszeichen

- 1: Röntgenaufnahme
- 2: Objekt
- 3: Röntgensystem
- 4: Röntgenquelle
- 5: Röntgendetektor
- 6: Tragarm
- 7: Blendenmatrix
- 8: gemeinsame Drehachse
- 9: erste Trajektorie
- 10: zweite Umlaufposition der Röntgenquelle
- 11: dritte Umlaufposition der Röntgenquelle
- 12: vierte Umlaufposition der Röntgenquelle
- 13: zweite Trajektorie
- 14: erste Umlaufpositionen der Blendematrix
- 15: zweite Umlaufpositionen der Blendematrix
- 16: dritte Umlaufpositionen der Blendematrix
- 17: vierte Umlaufpositionen der Blendematrix
- 18: Blendenelemente
- 19: Computer
- 20: Kabelverbindung
- 21: aufzunehmender Bereich
- 22: erster Blendenmatrixbereich
- 23: zweiter Blendenmatrixbereich
- 24: Röntgenstrahlen
- 25: Aufnahmeröntgenstrahlen
- 26: Kabelverbindung
- 27: Monitor
- 28: Tastatur
- 29: Maus
- 30: Cursor
- 31: Übersichtsaufnahme

## Patentansprüche

1. Verfahren zur Durchführung einer dreidimensionalen Röntgenaufnahme eines Objekts (2) mittels eines Röntgensystems (3), umfassend eine Röntgenquelle (4), einen Röntgendetektor (5), ein Datenverarbeitungssystem (19) und eine Blendenmatrix (7), wobei die Blendenmatrix (7) mehrere Blendenelemente (18) aufweist, die in ihren Röntgenabsorptionseigenschaften steuerbar sind, wobei mindestens ein erster aufzunehmender Bereich (21) des Objekts (2) festgelegt wird, wobei unter Berücksichtigung des ersten festgelegten aufzunehmenden Bereichs (21) Einstellungen für die einzelnen Blendenelemente (18) der Blendenmatrix (7) für verschiedene Umlaufpositionen (14, 15, 16, 17) geplant werden, wobei anschließend mehrere zweidimensionale Röntgenaufnahmen aus den geplanten Umlaufpositionen (14, 15, 16, 17) während mindestens eines partiellen Umlaufs (9) mit den geplanten Einstellungen der Blendenelemente (18) aufgenommen werden, wobei aus den einzelnen zweidimensionalen Röntgenaufnahmen die gesamte dreidimensionale Röntgenaufnahme (1) des ersten aufzunehmenden Bereichs (21) erzeugt wird, **dadurch gekennzeichnet, dass** zusätzlich zum ersten aufzunehmenden Bereich (21) mindestens ein zweiter aufzunehmender Bereich festgelegt wird, der vom ersten aufzunehmenden Bereich (21) räumlich abgetrennt ist, wobei die Blendenelemente (18) der Blendematrix (7) so angesteuert werden, dass der erste aufzunehmende Bereich (21) und der mindestens zweite aufzunehmende Bereich gleichzeitig aufgenommen werden, so dass zwei oder mehrere räumlich voneinander getrennte Bereiche gleichzeitig aufgenommen werden.

2. Verfahren nach Anspruch 1, **dadurch gekennzeichnet, dass** die aufzunehmenden Bereiche (21) in einer vor der Durchführung der dreidimensionalen Röntgenaufnahme durchgeführten Übersichtsaufnahme (31) festgelegt werden.

3. Verfahren nach Anspruch 2, **dadurch gekennzeichnet, dass** die Übersichtsaufnahme (31) eine zweidimensionale optische Aufnahme, eine dreidimensionale optische Aufnahme, eine zweidimensionale Röntgenaufnahme oder eine dreidimensionale Röntgenaufnahme ist.

4. Verfahren nach Anspruch 2 oder 3, **dadurch gekennzeichnet, dass** die aufzunehmenden Bereiche (21) manuell mittels eines Computers (19) unter Verwendung von Eingabemitteln (28, 29) durch einen Benutzer in der Übersichtsaufnahme (31) festgelegt werden.

5. Verfahren nach Anspruch 2 oder 3, **dadurch gekennzeichnet, dass** die aufzunehmenden Bereiche (21) automatisch mittels eines Computers (19) in der Übersichtsaufnahme (31) festgelegt werden, wobei bestimmte vordefinierte anatomische Bereiche (21) mittels eines computergestützten Suchalgorithmus erkannt werden.

6. Verfahren nach Anspruch 1, **dadurch gekennzeichnet, dass** die aufzunehmenden Bereiche (21) bei einer Vorauswahl festgelegt werden, wobei anhand von bekannten Lagebeziehungen der Röntgenquelle (4), des Röntgendetektors (5) und der Blendenmatrix (7) relativ zueinander und anhand von Abmessungen eines in einem Datenspeicher abgelegten Modells eines Patienten eine bestimmte anatomische Struktur des Musterkopfes als der aufzunehmende Bereich (21) festgelegt wird.

7. Verfahren nach einem der Ansprüche 1 bis 6, **dadurch gekennzeichnet, dass** die aufzunehmenden Bereiche (21) mindestens eine anatomische Struktur umfassen.

8. Verfahren nach Anspruch 7, **dadurch gekennzeichnet, dass** die anatomische Struktur ein Oberkiefer, ein Unterkiefer (21), ein rechtes Kiefergelenk und/oder ein linkes Kiefergelenk und/oder eine Teilstruktur dieser anatomischen Struktur ist.

9. Verfahren nach einem der Ansprüche 1 bis 8, **dadurch gekennzeichnet, dass** die Einstellungen für die Blendenelemente (18) der Blendenmatrix (7) während des mindestens partiellen Umlaufs an eine aufzunehmende anatomische Struktur (21) angepasst werden, wobei die aufzunehmende anatomische Struktur (21) in mindestens einer vorangehenden zweidimensionalen Röntgenaufnahme aus einer vorangehenden Umlaufposition (14) erkannt wird und anschließend die Einstellungen für die Blendenelemente (18) der Blendenmatrix (7) für eine nachfolgende Röntgenaufnahme aus einer nachfolgenden Umlaufposition (15) an die Abmessungen der erkannten anatomischen Struktur (21) aus der vorangehenden zweidimensionalen Röntgenaufnahme der vorangehenden Umlaufposition (14) angepasst wird.

10. Verfahren nach Anspruch 9, **dadurch gekennzeichnet, dass** die Anpassung der Einstellungen für die Blendenelemente (18) der Blendenmatrix (7) an die anatomische Struktur (21) schrittweise, Aufnahme für Aufnahme, für jede Umlaufposition (14, 15, 16, 17) erfolgt.

11. Verfahren nach einem der Ansprüche 1 bis 10, **dadurch gekennzeichnet, dass** die Blendenmatrix (7) eine Anordnung von in einer Ebene angeordneten Blendenelementen (18) ist.

12. Verfahren nach einem der Ansprüche 1 bis 10, **dadurch gekennzeichnet, dass** die Blendenmatrix eine zeilenförmige Anordnung von in einer Reihe angeordneten Blendenelementen ist.

## Claims

1. Method for capturing a three-dimensional X-ray image of an object (2) by means of an X-ray system (3) comprising an X-ray source (4), an X-ray detector (5), a data processing system (19) and a shutter matrix (7), wherein the shutter matrix (7) comprises a plurality of shutter elements (18), the X-ray absorption properties of which can be controlled, at least one first region (21) of the object (2) to be captured is defined, wherein settings for the individual shutter elements (18) of the shutter matrix (7) are planned for different revolution positions (14, 15, 16, 17) taking into account the first defined region to be captured (21), wherein a plurality of two-dimensional X-ray images is subsequently captured from the planned revolution positions (14, 15, 16, 17) using the planned settings of the shutter elements (18) during at least one partial revolution (9), wherein the overall three-dimensional X-ray image (1) of the first region (21) to be captured is generated from the individual two-dimensional X-ray images, **characterized in that**, in addition to the first region (21) to be captured, at least one second region to be captured is defined which is spatially separated from the first region (21) to be captured, wherein the shutter elements (18) of the shutter matrix (7) are controlled such that the first region (21) to be captured and the at least second region to be captured are captured at the same time, so that two or more spatially separated regions are captured at the same time.

2. Method according to Claim 1, **characterized in that** the regions (21) to be captured are defined in an overview image (31) taken prior to capturing the three-dimensional X-ray image.

3. Method according to Claim 2, **characterized in that** the overview image (31) is a two-dimensional optical image, a three-dimensional optical image, a two-dimensional X-ray image or a three-dimensional X-ray image.

4. Method according to Claim 2 or 3, **characterized in that** the regions (21) to be captured are manually defined in the overview image (31) by a user with the aid of a computer (19) and using input means (28, 29).

5. Method according to Claim 2 or 3, **characterized in that** the regions (21) to be captured are automatically defined in the overview image (31) by means of a computer (19), wherein specific predefined anatomical regions (21) are identified using a computer-assisted search algorithm.

6. Method according to Claim 1, **characterized in that** the regions (21) to be captured are defined in a preselection, wherein, on the basis of known positional relationships of the X-ray source (4), the X-ray detector (5) and the shutter matrix (7) relative to one another and on the basis of dimensions of a model of a patient stored in a data memory, a specific anatomical structure of the model head is defined as the region (21) to be captured.

7. Method according to any one of Claims 1 to 6, **characterized in that** the regions (21) to be captured include at least one anatomical structure.

8. Method according to Claim 7, **characterized in that** the anatomical structure is an upper jaw, a lower jaw (21), a right temporomandibular joint and/or a left temporomandibular joint and/or a partial structure of said anatomical structure.

9. Method according to any one of Claims 1 to 8, **characterized in that** the settings for the shutter elements (18) of the shutter matrix (7) are adjusted to an anatomical structure (21) to be captured during the at least partial revolution, wherein the anatomical structure (21) to be captured is identified in at least one preceding two-dimensional X-ray image from a preceding revolution position (14), after which the settings for the shutter elements (18) of the shutter matrix (7) are adjusted for a subsequent X-ray image from a subsequent revolution position (15) to the dimensions of the identified anatomical structure (21) from the preceding two-dimensional X-ray image of the preceding revolution position (14).

10. Method according to Claim 9, **characterized in that** the adjustment of the settings for the shutter elements (18) of the shutter matrix (7) to the anatomical structure (21) takes place incrementally, image by image, for each revolution position (14, 15, 16, 17).

11. Method according to any one of Claims 1 to 10, **characterized in that** the shutter matrix (7) is an arrangement of shutter elements (18) arranged in a plane.

12. Method according to any one of Claims 1 to 10, **characterized in that** the shutter matrix is a linear arrangement of shutter elements arranged in a row.

## Revendications

1. Procédé permettant d'effectuer une radiographie tridimensionnelle d'un objet (2) au moyen d'un système radiologique (3) comprenant une source de rayons X (4), un détecteur de rayons X (5), un système de traitement de données (19) et une matrice écran (7), ladite matrice écran (7) présentant plusieurs éléments écrans (18) dont les propriétés d'absorption des rayons X peuvent être régulées ; au moins une première zone à radiographier (21) de l'objet (2) étant déterminée ; des réglages des différents éléments écrans (18) de la matrice écran (7) étant programmés pour différentes positions périphériques (14, 15, 16, 17), compte tenu de la première zone à radiographier (21) définie, plusieurs radiographies bidimensionnelles étant ensuite effectuées à partir des positions périphériques programmées (14, 15, 16, 17) pendant au moins une rotation partielle (9) avec les réglages programmés des éléments écrans (18), une radiographie tridimensionnelle complète (1) de la première zone à radiographier (21) étant produite à partir des différentes radiographies bidimensionnelles ; **caractérisé en ce que**, en plus de la première zone à radiographier (21), il est déterminé au moins une deuxième zone à radiographier qui est séparée spatialement de la première zone à radiographier (21), lesdits éléments écrans (18) de la matrice écran (7) étant commandés de sorte que la première zone à radiographier (21) et au moins la deuxième zone à radiographier soient radiographiées en même temps, si bien que deux zones ou plus, séparées les unes des autres, sont radiographiées simultanément.

2. Procédé selon la revendication 1, **caractérisé en ce que** les zones à radiographier (21) sont déterminées dans une prise de vue générale (31) réalisée avant la réalisation des radiographies tridimensionnelles.

3. Procédé selon la revendication 2, **caractérisé en ce que** la prise de vue générale (31) est une prise de vue optique bidimensionnelle, une prise de vue optique tridimensionnelle, une radiographie bidimensionnelle ou une radiographie tridimensionnelle.

4. Procédé selon la revendication 2 ou 3, **caractérisé en ce que** les zones à radiographier (21) sont déterminées manuellement par un utilisateur dans la prise de vue générale (31) au moyen d'un ordinateur (19) par l'intermédiaire de moyens d'entrée (28, 29).

5. Procédé selon la revendication 2 ou 3, **caractérisé en ce que** les zones à radiographier (21) sont déterminées automatiquement au moyen d'un ordinateur (19) dans la prise de vue générale (31), des zones anatomiques prédéfinies spécifiques (21) étant reconnues au moyen d'un algorithme de recherche informatique.

6. Procédé selon la revendication 1, **caractérisé en ce que** les zones à radiographier (21) sont déterminées lors d'une présélection et dans lequel, à l'aide de relations de position connues des sources de rayons X (4), du détecteur de rayons X (5) et de la matrice écran (7) les uns par rapport aux autres et à l'aide des dimensions d'un modèle d'un patient mémorisé dans une mémoire de données, une structure anatomique définie de la tête modèle est déterminée comme zone à radiographier (21).

7. Procédé selon une des revendications 1 à 6, **caractérisé en ce que** les zones à radiographier (21) comprennent au moins une structure anatomique.

8. Procédé selon la revendication 7, **caractérisé en ce que** la structure anatomique est une mâchoire supérieure, une mâchoire inférieure (21), une articulation droite de la mâchoire et/ou une articulation gauche de la mâchoire et/ou une structure partielle de cette structure anatomique.

9. Procédé selon une des revendications 1 à 8, **caractérisé en ce que** les réglages concernant les éléments écrans (18) de la matrice écran (7) sont adaptés, pendant la rotation au moins partielle, à une structure anatomique à radiographier (21), ladite structure anatomique à radiographier (21) étant reconnue dans au moins une radiographie bidimensionnelle précédente à partir d'une position périphérique précédente (14), après quoi les réglages concernant les éléments écrans (18) de la matrice écran (7) sont adaptés pour une radiographie suivante à partir d'une position périphérique suivante (15) aux dimensions de la structure anatomique reconnue (21) à partir de la radiographie bidimensionnelle précédente de la position périphérique précédente (14).

10. Procédé selon la revendication 9, **caractérisé en ce que** l'adaptation des réglages concernant les éléments écrans (18) de la matrice écran (7) à la structure anatomique (21) est effectuée par étape, prise de vue par prise de vue, pour chaque position périphérique (14, 15, 16, 17).

11. Procédé selon une des revendications 1 à 10, **caractérisé en ce que** la matrice écran (7) est un agencement d'éléments écrans (18) disposés dans un plan.

12. Procédé selon une des revendications 1 à 10, **caractérisé en ce que** la matrice écran est un agencement linéaire d'éléments écrans disposés dans une rangée.
